# EUROPEAN PATENT APPLICATION

(11) **EP 2 123 280 A1**
(43) Date of publication of application: **25.11.2009**
(21) Application number: 08720364.2
(22) Date of filing: 07.03.2008
(51) Int. Cl.: A61K 31/573, A61K 31/50, A61P 37/02, A61P 43/00, C07D 237/06

(54) **AGENT FOR PREVENTION AND/OR TREATMENT OF SYSTEMIC LUPUS ERYTHEMATOSUS**

(30) Priority: 09.03.2007 JP 2007060090
(71) Applicant: Kowa Co., Ltd., Nagoya-shi, Aichi 460-8625 (JP)
(72) Inventor: TABUNOKI, Yuichiro, Tokyo 189-0022 (JP)
(74) Representative: Blodig, Wolfgang
(86) International application number: PCT/JP2008/000478
(87) International publication number: WO 2008/111295

(57) **Abstract**

Provided is an agent for prevention and/or treatment of systemic lupus erythematosus, which comprises, in combination, 2-benzyl-5-(4-chlorophenyl)-6-[4-(methylthio)phenyl]-2H-pyrida zine-3-one or a solvate thereof and a corticosteroid. The pharmaceutical agent of the present invention is orally administrable, has fewer adverse effects, exhibits an excellent effect of suppressing the symptoms associated with SLE, and thus is useful for prevention and treatment of SLE.

## Description

### Technical Field

The present invention relates to an agent for prevention and/or treatment of systemic lupus erythematosus.

### Background Art

Systemic lupus erythematosus (SLE) is an autoimmune disease characterized by systemic inflammatory lesions due to tissue deposition of an immune complex such as a DNA-anti-DNA antibody. The symptoms of SLE are characterized by being alleviated through treatment but following chronic courses with repetition of remission and exacerbation in many cases. The incidence of SLE in the general population is estimated to be 10 to 100 people for each 100,000 people. However, the incidence rises to 2 to 4% when a patient suffering from SLE is present in the first-degree relatives, and rises to 25% in identical twins. Therefore, SLE is considered to be a disease that is highly affected by genetic factors. There is also known that although the ratio of male to female of the incidence of SLE is 1:9, the ratio of male to female becomes substantially 1:1 when females in fertile ages are excluded, and SLE occurs more commonly in young women in their 20's to 40's (see Non-patent Document 1).

On the other hand, as environmental factors, there are exemplified ultraviolet radiation, viral infection, injuries, operations, pregnancy, childbearing, and medicament treatment. It is considered that most of the environmental factors cause tissue damage, cell destruction, and release of a large amount of nuclei and cell components into the blood, which elicits immune response against autoantigens (see Non-patent Document 1).

The symptoms of SLE, as indicated by its disease name, are known to appear as various pathologic conditions in the whole body, and may be classified broadly into the following 10 categories (see Non-patent Document 2).
(1) Systemic symptoms: Systemic malaise, fatigability, and fever occur initially in many cases.
(2) Skin and mucous symptoms: Butterfly erythema and discoid rush are characteristic symptoms. Butterfly erythema has a feature of spreading ranging from cheek to nasal bridge. The exposure to sunlight exacerbates the symptoms. In skin biopsies, IgG deposition is confirmed in the dermoepidermal junction (positive for lupus band test). Discoid rash is often observed in the facial surface, auricle, head, dorsum of joint, and the like, initially is an erythema, but eventually causesinduration,cornification,scars,and atrophy. In addition, chilblain lupus, loss of scalp hair, and supersensitivity to sunlight are also characteristic of the symptoms. A painless ulcer appears in the mouth and nasopharynx in some cases.
(3) Muscular and articular symptoms: Muscular pain and articular pain are often observed in an acute stage. Arthritis is also observed, but is characterized by not being accompanied by osteoclasis.
(4) Renal symptoms: Glomerulonephritis (lupus nephritis) appears in about half of cases, and becomes severe if left untreated. In an acute stage, proteinuria is observed, and in urinary sediment, a large number of red blood cells, white blood cells, casts, and the like appear.
(5) Nervous symptoms: The case where central nervous symptoms are presented is serious (CNS lupus). There are often observed spirit symptoms such as depression, disorientation, and delusion, convulsions, and cerebrovascular disorder. Meningitis, encephalitis, and cranial neuropathy are also less frequently observed.
(6) Cardiovascular symptoms: Pericarditis is often observed. When myocarditis occurs, tachycardia and arrhythmia appear. Generally, valvular lesions have no symptoms, but cause mild aortic valve insufficiency and mitral insufficiency in some cases. Further, when thrombophlebitis occurs repeatedly, it is suspected to accompany antiphospholipid antibody syndrome.
(7) Pulmonary symptoms: Pleurisy is of ten observed in an acute stage. In addition, caution should be exercised in interstitial pneumonitis, cell hemorrhage, and pulmonary hypertension because they are pathologic conditions suggesting poor prognosis.
(8) Gastrointestinal symptoms: Mesenteric vasculitis and lupus peritonitis both accompanied by abdominal pain may occur in some cases.
(9) Hematopoietic symptoms: Hemolytic anemia is often observed, and diagnosed from the findings of the increase in reticulocyte and the decrease in haptoglobin, for example. The decrease in white blood cells and the decrease in platelets are also often observed, which have been considered to be due to cell destruction in periphery.
(10) Others: Lymphadenopathy is often observed in an acute stage.

As mentioned above, SLE exhibits a wide variety of symptoms, and hence the treatment of SLE includes combinations of the following elements (see Non-patent Document 3):
(1) Potent immunosuppressive therapy for autoimmunity of SLE;
(2) Treatment of systemic multiorgan lesions of SLE;
(3) Long-term treatment regimen for SLE, which is a chronic disease, in view of prognoses of life and organs;
(4) Consideration for young women (in particular, pregnancy and osteoporosis);
(5) Consideration for adverse effects of a corticosteroid, which is a key drug; and
(6) Consideration for environmental factors (training and mentoring of patients).

The central agent for treatment of SLE is still a corticosteroid such as prednisolone. The corticosteroid has an anti-inflammatory action and an immunosuppressive action, and is considered to inhibit local inflammation exhibiting organ lesions, and besides, to suppress the production of autoantibodies and inhibit autoreactive lymphocytes, thereby providing effects of treating diseases. On the other hand, when the resistance against the corticosteroid occurs and when a severe adverse effect occurs, the administration of an immunosuppresant is taken into account. As the immunosuppresant, azathioprine, which is a purine metabolism inhibitor, and cyclophosphamide, which is a DNA-alkylating agent, are often used through oral administration. Further, it has been reported that the oral administration of mizoribine, which is an inhibitor of inosine monophosphate (IMP) dehydrogenase that is a rate-determining enzyme in a purine biosynthetic pathway, is effective for lupus nephritis (see Non-patent Document 3). It has also been reported that an effect obtained by using immunosuppresant and a corticosteroid in combination is useful (see Non-patent Document 4).

On the other hand, in recent years, biologics targeting B cells such as CD20 and BAFF have been developed (see Non-patent Document 5) and have been expected as novel agents for treatment of SLE. In addition, it has been reported that there is a correlation between interferon-inducible genes and activity of SLE (see Non-patent Document 6). Thus, the interferon-inducible genes have attracted attention as novel targets for treatment.

The increase in production of interleukin-1β (IL-1β), which is an inflammatory cytokine, is recognized in a number of diseases such as rheumatoid arthritis, osteoarthritis, osteoporosis, inflammatory bowel disease, immune deficiency syndrome, sepsis, hepatitis, nephritis, ischemic disease, insulin-dependent diabetes mellitus, arteriosclerosis, Parkinson's disease, Alzheimer's disease, and leukemia. In particular, IL-1β is known to cause articular destruction which is very similar to rheumatoid arthritis following its intraarticular injection in animals. Thus, IL-1β inhibitors have been researched and developed as agents for treatment of inflammatory diseases, and there are known substances derived from biogenic components such as IL-1 receptor antagonists (see Non-patent Document 7) and low-molecular compounds such as T-614 (see Non-patent Document 8) and 2-benzyl-5-(4-chlorophenyl)-6-[4-(methylthio)phenyl]-2H-pyridazine-3-one (see Patent Document 1).

The pathologic condition of SLE also has an aspect of an inflammatory disease to be induced by autoimmunity. Macrophages play a key role in the development of lupus nephritis, which is a particularly typical symptom of SLE, and cytokines involved in the activation of the macrophages and cytokines produced by the activation have attracted attention (see Non-patent Document 9). Of those, it has been suggested that the inhibition of IFN-γ (see Non-patent Document 10) and the inhibition of TNF-α (see Non-patent Document 11) or the administration of anakinra, which is an IL-1 receptor antagonist (see Non-patent Document 12), lead to an improvement in the pathologic condition of SLE.
However, there remains unknown an effect obtained by using a corticosteroid and an IL-1β inhibitor in combination on SLE.
[Patent Document 1] WO 99/25697
[Non-patent Document 1] Nippon Rinsho 63, Suppl 5, pp. 247-252, 2005
[Non-patent Document 2] "Japan Intractable Diseases Information Center", [online], Ministry of Health, Labour and Welfare, [searched on 28 February, 2007], Internet <URL: http://www.nanbyou.or.jp/sikkan/063_i.htm>
[Non-patent Document 3] Nippon Rinsho 63, Suppl 5, pp. 253-259, 2005
[Non-patent Document 4] Pediatr. Int. 44, pp. 199-204, 2002
[Non-patent Document 5] Expert Opin. Ther. Targets 10, pp. 803-815, 2006
[Non-patent Document 6] Arthritis & Rheumatism 50, pp. 3958-3967, 2004
[Non-patent Document 7] Ann. Rheum. Dis. 59 (suppl I) pp. 103-108, 2000
[Non-patent Document 8] J. Pharmacobio-Dyn. 15, pp. 649-655, 1992
[Non-patent Document 9] Lupus 13, pp. 344-347, 2004
[Non-patent Document 10] J. Exp. Med. 166, pp. 798-803, 1987
[Non-patent Document 11] Arthritis & Rheumatism 50, pp. 3161-3169, 2004
[Non-patent Document 12] Ann. Rheum. Dis. 64, pp. 630-633, 2005

### Disclosure of the Invention

### Problem to be solved by the Invention

It is an object of the present invention to provide a drug having an excellent effect of preventing and/or treating systemic lupus erythematosus (SLE).

### Means for solving the Problems

The inventors of the present invention have intensively studied in view of such actual circumstances. As a result, the inventors have found that a synergistically excellent effect of suppressing the symptoms of SLE, and in particular, the expression of SLE-associated genes in the kidney is obtained by administering 2-benzyl-5-(4-chlorophenyl)-6-[4-(methylthio)phenyl]-2H-pyrida zine-3-one, which is an IL-1β inhibitor, and a corticosteroid in combination, and the effect is so potent that the degree of the effect is unpredictable from an effect which is provided when each drug is administered alone. Thus, the inventors have completed the present invention.

That is, the present invention provides an agent for prevention and/or treatment of systemic lupus erythematosus, which comprises, in combination, 2-benzyl-5-(4-chlorophenyl)-6-[4-(methylthio)phenyl]-2H-pyridazine-3-one or a solvate thereof and a corticosteroid.
Further, the present invention provides the use of 2-benzyl-5-(4-chlorophenyl)-6-[4-(methylthio)phenyl]-2H-pyrida zine-3-one or a solvate thereof and a corticosteroid for production of an agent for prevention and/or treatment of systemic lupus erythematosus.
Still further, the present invention provides a combination of 2-benzyl-5-(4-chlorophenyl)-6-[4-(methylthio)phenyl]-2H-pyridazine-3-one or a solvate thereof and a corticosteroid for prevention and/or treatment of systemic lupus erythematosus.
Yet still further, the present invention provides a method for preventing and/or treating systemic lupus erythematosus, characterized by administering 2-benzyl-5-(4-chlorophenyl)-6-[4- (methylthio) phenyl] -2H-pyridazin-3-one or a solvate thereof and a corticosteroid in combination.

### Effects of the Invention

The agent for prevention and/or treatment of SLE of the present invention is orally administrable, has fewer adverse effects, exhibits an excellent effect of suppressing the symptoms associated with SLE, and thus is useful for prevention and treatment of SLE.

### Brief Description of the Drawings

FIG. 1 is a graph illustrating an effect of a drug on an mRNA expression level of IFN-γ in the kidney of an SLE model mouse.
FIG. 2 is a graph illustrating an effect of a drug on an mRNA expression level of TNF-α in the kidney of an SLE model mouse.
FIG. 3 is a graph illustrating an effect of a drug on an mRNA expression level of IL-1β in the kidney of an SLE model mouse.
FIG. 4 is a graph illustrating an effect of a drug on an mRNA expression level of BAFF in the kidney of an SLE model mouse.
FIG. 5 is a graph illustrating an effect of a drug on an mRNA expression level of IFIT1 in the kidney of an SLE model mouse.
FIG. 6 is a graph illustrating an effect of a drug on an mRNA expression level of IP-10 in the kidney of an SLE model mouse.

### Best Mode for carrying out the Invention

2-Benzyl-5-(4-chlorophenyl)-6-[4-(methylthio) phenyl] -2H-pyridazine-3-one to be used in an agent for prevention and/or treatment of SLE of the present invention may be produced by the method described in WO 99/25697 or other similar methods, for example.

Further,2-benzyl-5-(4-chlorophenyl)-6-[4-(methylthio)phenyl]-2H-pyridazine-3-one may be present in a form of a solvate typified by a hydrate, and the present invention also encompasses such a solvate.

The corticosteroids to be used in the agent for prevention and/or treatment of SLE of the present invention involve all of the corticosteroids whose potencies are expressed in terms of prednisolone. Here, an example of the corticosteroids includes a compound that has a steroid skeleton and has a glucocorticoid action and/or a mineralocorticoid action. Further, a hydrate of those compounds and salts thereof and an ester of those compounds and salts thereof, and a solvate with a pharmaceutically acceptable solvent are encompassed. In addition, when those compounds have asymmetric carbon atoms, and when those compounds have unsaturated bonds and their stereoisomers are present, all those isomers are encompassed.

Examples of the corticosteroids suitable for the present invention include prednisolone, methyl prednisolone, betamethasone, and dexamethasone palmitate, and in particular, prednisolone is preferred. Those corticosteroids are available as commercially-available products such as reagents or pharmaceutical agents. For example, prednisolone is available from Sigma-Aldrich Co.

As shown in the examples below, in an evaluation system using NZB/WF1 mice widely known as an animal model of systemic lupus erythematosus, when 2-benzyl-5-(4-chlorophenyl)-6-[4-(methylthio)phenyl]-2H-pyrida zine-3-one or a solvate thereof is used alone, there was not confirmed a significant action of suppressing the expression of SLE-associated genes in the kidney, while when 2-benzyl-5-(4-chlorophenyl)-6-[4-(methylthio)phenyl]-2H-pyridazine-3-one or a solvate thereof and an corticosteroid have been administered in combination, the expression of SLE-associated genes in the kidney has been highly reduced. Therefore, according to the present invention, systemic lupus erythematosus may be prevented and/or treated.

The administration route of the drug to be used in the agent for prevention and/or treatment of SLE of the present invention is not particularly limited, and may be appropriately selected depending on the purpose of treatment. For example, there are mentioned oral administration using tablets, capsules, granules, film-coated agents, powders, syrups, or the like, or parenteral administration using injections, suppositories, inhalants, percutaneous absorption agents, eye drops, nasal drops, or the like, and in particular, oral administration is preferred.

In pharmaceutical formulations suitable for those dosage forms, there may be appropriately used in combination with pharmaceutically acceptable carriers, for example, excipients and fillers such as starches, lactose, sucrose, mannitol, and silicic acid; disintegrants such as agar, calcium carbonate, potato or tapioca starch, alginic acid, and particular complex silicates; binders such as hydroxypropylmethylcellulose, alginates, gelatin, polyvinylpyrrolidone, sucrose, and gum arabic; lubricants such as talc, calcium stearate, magnesium stearate, solid polyethylene glycols, sodium lauryl sulfate, or mixtures thereof; diluents such as lactose and corn starch; buffers such as organic acids (for example, citric acid, tartaric acid, and lactic acid), inorganic acids (for example, phosphoric acid and hydrochloric acid), alkali hydroxides (for example, sodium hydroxide and potassium hydroxide), and amines (for example, triethanolamine, diethanolamine, and diisopropanolamine) ; antiseptics such as paraoxybenzoic acid esters and benzalkonium chloride; emulsifiers such as anionic surfactants (for example, calcium stearate, magnesium stearate, and sodium lauryl sulfate), cationic surfactants (for example, benzalkonium chloride, benzethonium chloride, and cetylpyridinium chloride), nonionic surfactants (for example, glyceryl monostearate, sucrose fatty acid esters, polyoxyethylene hardened castor oil, polyoxyethylene sorbitan fatty acid esters, polyoxyethylene fatty acid esters, and polyoxyethylene alkyl ethers); stabilizers such as sodium sulfite, sodium bisulfite, dibutylhydroxytoluene, butylhydroxyanisole, and edetic acid. In addition, corrigents, dispersants, preservatives, flavors, and the like may also be appropriately used in combination, as required.

The usage form of the drug of the present invention is not particularly limited as long as 2-benzyl-5-(4-chlorophenyl)-6-[4-(methylthio)phenyl]-2H-pyrida zine-3-one or a solvate thereof and a corticosteroid are used in combination, and the administration of both drugs provides a synergistic effect of suppressing the symptoms of SLE. 2-benzyl-5-(4-chlorophenyl)-6-[4-(methylthio)phenyl]-2H-pyrida zine-3-one or a solvate thereof and a corticosteroid may be simultaneously administered or may be separately administered at some interval. That is, with regard to 2-benzyl-5-(4-chlorophenyl)-6-[4-(methylthio)phenyl]-2H-pyrida zine-3-one or a solvate thereof and a corticosteroid, both drugs may be formulated into a single formulation, or both drugs may be separately formulated and used as a set (kit).

In the present invention, when both drugs are administered as a single formulation, the compounding ratio of 2-benzyl-5-(4-chlorophenyl)-6-[4-(methylthio)phenyl]-2H-pyrida zine-3-one or a solvate thereof to a corticosteroid is 100:1 to 1:100 by mass, and further, the ratio is preferably in the range of 10:1 to 1:10 from the viewpoint of achieving a particularly excellent synergistic effect.

Further, in the present invention, when both drugs are formulated separately, a formulation containing 2-benzyl-5-(4-chlorophenyl)-6-[4-(methylthio)phenyl]-2H-pyrida zine-3-one or a solvate thereof is provided as an agent for prevention and/or treatment of systemic lupus erythematosus that is administered in combination with a corticosteroid, while a formulation containing an corticosteroid is provided as an agent for prevention and/or treatment of systemic lupus erythematosus that is administered in combination with 2-benzyl-5-(4-chlorophenyl)-6-[4-(methylthio)phenyl]-2H-pyrida zine-3-one or a solvate thereof. The dosage form of both drugs may be the same or different from each other. Further, the number of administering each component may be different.

In the present invention, the dose of 2-benzyl-5-(4-chlorophenyl)-6-[4-(methylthio)phenyl]-2H-pyrida zine-3-one or a solvate thereof is appropriately selected based on the body weight, age, gender, symptoms, and the like of the patients. Generally, in the case of an adult, the dose may be 2 to 320 mg and preferably 4 to 160 mg, per day. Further, the dosage of the corticosteroid may increase or decrease depending on the symptoms, and in the case of an adult, the dose is preferably 10 to 200 mg per day in terms of prednisolone. In addition, the administration may be carried out once a day or twice or more a day with the dose being divided accordingly.

### Examples

Hereinafter, the present invention is more specifically described by way of examples, but the present invention is not limited to those examples.

### Example 1

Actions of suppressing the expression of disease-associated genes in the kidney by the administration of 2-benzyl-5-(4-chlorophenyl)-6-[4-(methylthio)phenyl]-2H-pyrida zine-3-one (hereinafter referred to as Drug A) and prednisolone (hereinafter referred to as Drug B) in combination and the administration of each drug alone were evaluated by using NZB×NZW (NZB/W)F1 mice widely known as spontaneous SLE models.

Female NZB/WF1 mice (Japan SLC, Inc.) were used as test animals.

Thebodyweightof 20-week-old NZB/WF1 mice was measured.
By using the body weight as an index, the mice were divided into groups through one-parameter-based block randomization so that each group becomes uniform.

Drug administration was conducted from the day after the grouping to 10 weeks thereafter. To a group for sole administration of Drug A, a dose of 30mg/kg was orally administered, twice a day, in the morning (9:00 to 11:00) and in the evening (15:30 to 17:30). Further, to a group for sole administration of Drug B, a dose of 3 mg/kg was orally administered, once a day, in the daytime (11:30 to 13:30). On the other hand, to a group for combined administration of Drug A and Drug B, 30 mg/kg of Drug A was orally administered in the morning (9: 00 to 11:00) and in the evening (15:30 to 17:30), and 3 mg/kg of Drug B was orally administered in the daytime (11:30 to 13:30).

10 weeks after the grouping (at the time of 30 weeks old), the kidney was excised from each individual and horizontally divided into two portions. After that, the kidney was subjected to RNase inactivation treatment that involves immersing the kidney in RNAlater (manufactured by QIAGEN GmbH) at 4°C for 24 hours. Then, the kidney divided into two portions was further divided into three portions, a front-end portion, a central portion, and a back-end portion. Total RNA was extracted from the central portion including hilum renalis, medulla, and cortex by using RNeasy kit (manufactured by QIAGEN GmbH) and QiaShredder (manufactured by QIAGEN GmbH). The mRNA expression levels of SLE-associated genes (IFN-γ (probe No.: Mm00801778_m1), TNF-α (probe No.: Mm00443258_m1), IL-1β (probe No.: Mm00434228_m1), BAFF (probe No.: Mm00446347_m1), IFIT1 (probe No.: Mm00515153_m1), and IP-10 (probe No.: Mm00445235_m1)) were determined by using ABI PRISM (registered trademark) 7900 Sequence Detection System (manufactured by Applied Biosystems, Inc.) and TaqMan (registered trademark) Gene Expression Assays (manufactured by Applied Biosystems, Inc.) by Real-Time RT-PCR method. It should be noted that those mentioned above were selected as evaluation targets: IFN-γ as a macrophage activating factor; TNF-α and IL-1β as inflammatory cytokines produced by activated macrophages; BAFF as a factor involved in B cells; and IFIT1 and IP-10 as an interferon-inducible gene group.

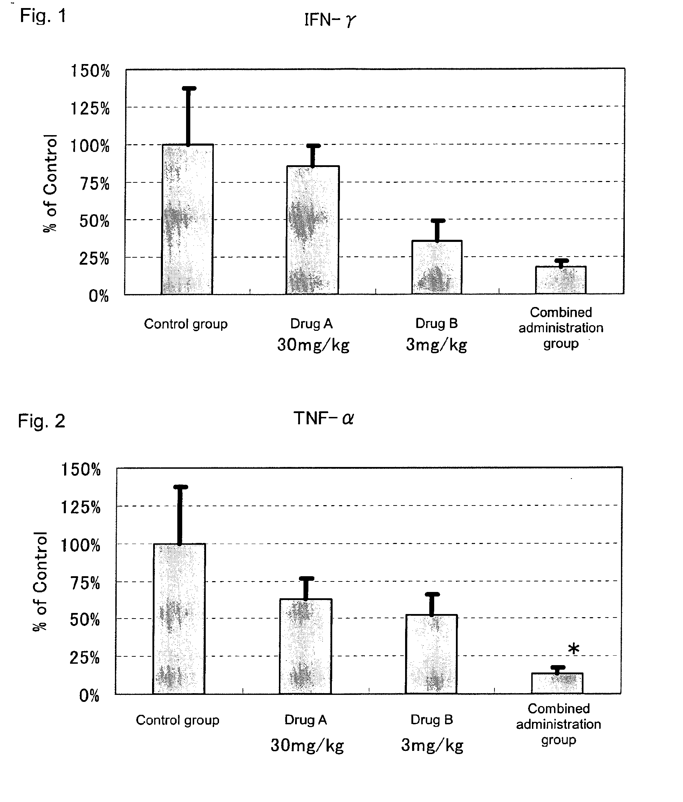

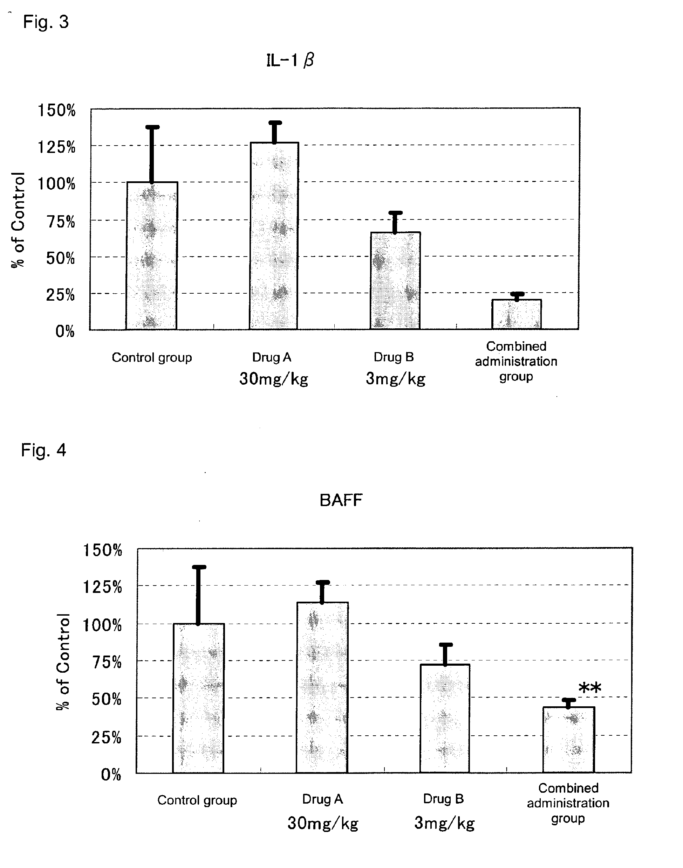

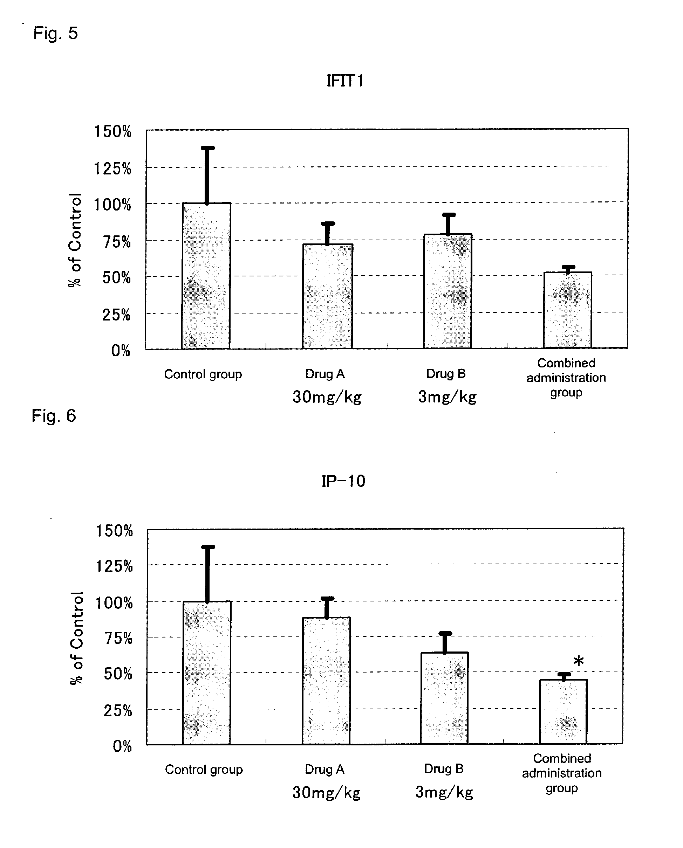

Tables 1 to 6 and FIGS. 1 to 6 each show the mRNA expression levels of IFN-γ (Table 1, FIG. 1), TNF-α (Table 2, FIG. 2), IL-1β (Table 3, FIG. 3), BAFF (Table 4, FIG. 4), IFIT1 (Table 5, FIG. 5), and IP-10 (Table 6, FIG. 6) in the kidney in a control group, a group for sole administration of 30 mg/kg of Drug A, a group for sole administration of 3 mg/kg of Drug B, and a group for combined administration of both Drugs. The respective values were normalized to the mean for the control group that was defined as 100%. The mRNA expression level is expressed as mean ± standard error of 10 to 11 mice in each group. Further, in the evaluation, statistical analysis was carried out by Dunnett' s multiple comparison test (**: p<0.01, * : p<0.05; both of which represent p-values for the control group).

**Table 1**

| Test drug | IFN-γ mRNA expression level(%) | Relative index |
|---|---|---|
| Control group | 100.0±37.8 | 1.000 |
| Group for sole administration of 30 mg/kg of Drug A | 85.7±27.1 | 0.857 |
| Group for sole administration of 3 mg/kg of Drug B | 36.0±13.1 | 0.360 |
| Group for combined administration of Drug A and Drug B | 18.2±4.7 | 0.182 |

| | | |
|---|---|---|
| Note 1) Product of relative indices of groups for sole administration: 0.857×0.360=0.309 Note 2) The mRNA expression level of is expressed as mean ± standard error of 10 to 11 mice in each group. | | |

**Table 2**

| Test drug | TNF-α mRNA expression level(%) | Relative index |
|---|---|---|
| Control group | 100.0±37.4 | 1.000 |
| Group for sole administration of 30 mg/kg of Drug A | 63.4±13.5 | 0.634 |
| Group for sole administration of 3 mg/kg of Drug B | 52.2±13.2 | 0.522 |
| Group for combined administration of Drug A and Drug B | 13.5±3.9 | 0.135 |

| | | |
|---|---|---|
| Note 1) Product of relative indices of groups for sole administration: 0.634×0.522=0.331 Note 2) The mRNA expression level is expressed as mean ± standard error of 10 to 11 mice in each group. | | |

**Table 3**

| Test drug | IL-1β mRNA expression level(%) | Relative index |
|---|---|---|
| Control group | 100.0±38.6 | 1.000 |
| Group for sole administration of 30 mg/kg of Drug A | 126.6±50.3 | 1.266 |
| Group for sole administration of 3 mg/kg of Drug B | 66.1±21.9 | 0.661 |
| Group for combined administration of Drug A and Drug B | 20.3±5.8 | 0.203 |

| | | |
|---|---|---|
| Note 1) Product of relative indices of groups for sole administration: 1.266×0..61=0.836 Note 2) The expression amount of mRNA is expressed as mean ± standard error of 10 to 11 mice in each group. | | |

**Table 4**

| Test drug | BAFF mRNA expression level (%) | Relative index |
|---|---|---|
| Control group | 100.0±14.1 | 1.000 |
| Group for sole administration of 30 mg/kg of Drug A | 113.5±13.7 | 1.135 |
| Group for sole administration of 3 mg/kg of Drug B | 72.2±13.1 | 0.722 |
| Group for combined administration of Drug A and Drug B | 44.1±6.6 | 0.441 |

| | | |
|---|---|---|
| Note 1) Product of relative indices of groups for sole administration: 1.135×0.722=0.820 Note 2) The expression amount of mRNA is expressed as mean ± standard error of 10 to 11 mice in each group. | | |

**Table 5**

| Test drug | IFIT1 mRNA expression level(%) | Relative index |
|---|---|---|
| Control group | 100.0±12.7 | 1.000 |
| Group for sole administration of 30 mg/kg of Drug A | 72.1±15.6 | 0.721 |
| Group for sole administration of 3 mg/kg of Drug B | 78.6±15.0 | 0.786 |
| Group for combined administration of Drug A and Drug B | 51.7±19.4 | 0.517 |

| | | |
|---|---|---|
| Note 1) Product of relative indices of groups for sole administration: 0.721×0.786=0.566 Note 2) The expression amount of mRNA is expressed as mean ± standard error of 10 to 11 mice in each group. | | |

**Table 6**

| Test drug | IP-10 mRNA expression level(%) | Relative index |
|---|---|---|
| Control group | 100.0±10.8 | 1.000 |
| Group for sole administration of 30 mg/kg of Drug A | 88.3±18.1 | 0.883 |
| Group for sole administration of 3 mg/kg of Drug B | 63.5±9.9 | 0.635 |
| Group for combined administration of Drug A and Drug B | 44.7±15.8 | 0.447 |

| | | |
|---|---|---|
| Note 1) Product of relative indices of groups for sole administration: 0.883×0.635=0.566 Note 2) The expression amount of mRNA is expressed as mean ± standard error of 10 to 11 mice in each group. | | |

A dose of 30 mg/kg of Drug A exhibited no action on the expression of SLE-associated genes in the kidney of this model or exhibited only a tendency to slightly suppress the expression. On the other hand, a dose of 3 mg/kg of Drug B exhibited a tendency to suppress the expression of any gene. However, the combined administration of Drug A and Drug B resulted in significant decrease in the expression of SLE-associated genes in the kidney. Further, the relative index for the expression level of SLE-associated genes is smaller than the product of relative indexes of the groups for sole administration of each drug, and hence, a clear synergistic effect due to the combined use was observed. Thus, an effect of preventing and treating SLE obtained by using Drug A and Drug B in combination went far beyond the prediction made on the basis of the effect exhibited when each drug was used alone.

From the above results, the administration of 2-benzyl-5-(4-chlorophenyl)-6-[4-(methylthio)phenyl]-2H-pyrida zin-3-one or a solvate thereof and a corticosteroid in combination provides an excellent effect of preventing and treating systemic lupus erythematosus, which is too excellent to predict the provided effect on the basis of the effect exhibited when each drug is administered alone. Accordingly, both drugs administered in combination are recognized to be useful as an agent for prevention and/or treatment of systemic lupus erythematosus.

## Claims

1. An agent for prevention and/or treatment of systemic lupus erythematosus, which comprises, in combination, 2-benzyl-5-(4-chlorophenyl)-6-[4-(methylthio)phenyl]-2H-pyrida zine-3-one or a solvate thereof and a corticosteroid.

2. The agent for prevention and/or treatment of systemic lupus erythematosus according to Claim 1, wherein the corticosteroid is selected from prednisolone, methyl prednisolone, betamethasone, dexamethasone palmitate, and a solvate thereof.

3. The agent for prevention and/or treatment of systemic lupus erythematosus according to Claim 1, wherein the corticosteroid is prednisolone or a solvate thereof.

4. The agent for prevention and/or treatment of systemic lupus erythematosus according to any one of Claims 1 to 3, wherein a dosage form is an oral formulation.

5. Use of 2-benzyl-5-(4-chlorophenyl)-6-[4-(methylthio)phenyl]-2H-pyrida zine-3-one or a solvate thereof and a corticosteroid for production of an agent for prevention and/or treatment of systemic lupus erythematosus.

6. The use according to Claim 5, wherein the corticosteroid is selected from prednisolone, methyl prednisolone, betamethasone, dexamethasone palmitate, and a solvate thereof.

7. The use according to Claim 5, wherein the corticosteroid is prednisolone or a solvate thereof.

8. The use according to any one of Claims 5 to 7, wherein a dosage form is oral formulation.

9. A method for prevention and/or treatment of systemic lupus erythematosus, wherein 2-benzyl-5-(4-chlorophenyl)-6-[4-(methylthio)phenyl]-2H-pyrida zine-3-one or a solvate thereof and a corticosteroid are administrated in combination.

10. The method for prevention and/or treatment of systemic lupus erythematosus according to Claim 9, wherein the corticosteroid is selected from prednisolone, methyl prednisolone, betamethasone, dexamethasone palmitate, and a solvate thereof.

11. The method for prevention and/or treatment of systemic lupus erythematosus according to Claim 9, wherein the corticosteroid is prednisolone or a solvate thereof.

12. The method for prevention and/or treatment of systemic lupus erythematosus according to any one of Claims 9 to 11, wherein a means for administration comprises oral administration.
